# EUROPEAN PATENT APPLICATION

(11) **EP 4 151 222 A1**
(43) Date of publication of application: **22.03.2023**
(21) Application number: 21196927.4
(22) Date of filing: 15.09.2021
(51) Int. Cl.: A61K 31/702, A61K 36/185, A61P 25/00

(54) **COMPOSITION FOR OPTIMIZING UROLITHIN PRODUCTION IN A HUMAN SUBJECT**

(71) Applicant: Migalis ApS, 2950 Vedbæk (DK)
(72) Inventor: Giversen, Ina, 2000 Frederiksberg (DK); Jensen, Leif Helth, 6354 Vitznau (CH)
(74) Representative: AWA Denmark A/S

(57) **Abstract**

The present invention relates to a composition comprising 1) a source of ellagitannins and/or ellagic acid, such as a preparation *Punica granatum* L. and 2) one or more human milk oligosaccharides (HMOs). Also disclosed herein is a method of altering the metabotype of a human subject towards 1) favouring the relative production of urolithin A compared with other final urolithins and/or 2) increasing the production of urolithin A metabolites in the gut by administering said composition to said human subject, in particular a human subject of the mixed urolithin producer metabotype.

## Description

### TECHNICAL FIELD

The present invention relates to a composition comprising 1) a source of ellagitannins and/or ellagic acid such as a preparation of *Punica granatum* L. and 2) one or more human milk oligosaccharides (HMOs). Also disclosed herein is a method of optimizing the production of urolithins in the gut of a human subject, especially with a view to alter the relative amounts of produced final urolithins towards mainly urolithin A by administering said composition to the human subject.

### BACKGROUND OF THE INVENTION

Urolithins are gut microbiota-derived metabolites resulting from the catabolization of ellagitannins and/or ellagic acid by the gut microbiota. Rich sources of ellagitannins are pomegranate and certain fruits and nuts, such as raspberry, strawberry, blackberry and walnut.

Ellagitannins are hydrolyzed in the gut to release ellagic acid, which is further processed by the gut microbiota into urolithins through the loss of one of its two lactones and by successive removal of hydroxyl groups.

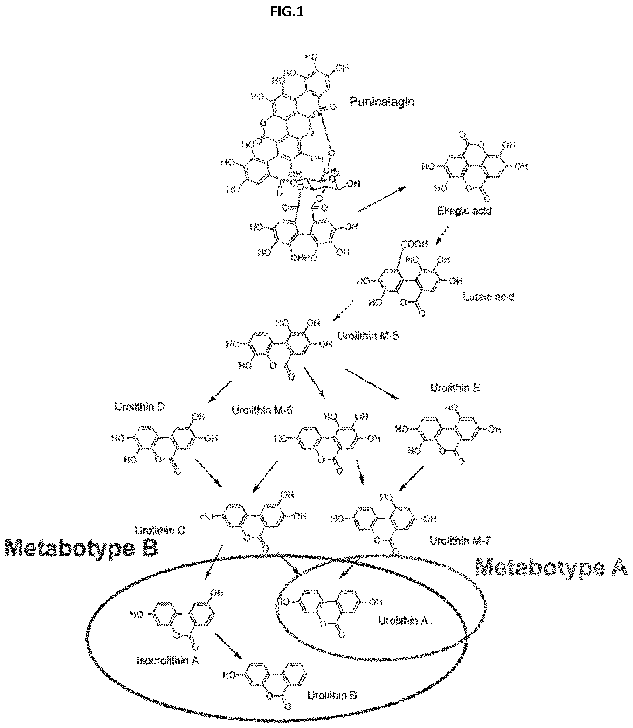

In humans, urolithin A, urolithin B and isourolithin A are the measurable final metabolites of the catabolic conversion of ellagitannins and/or ellagic acid (see Figure 1). Urolithin A is the main metabolite observed in human plasma and urine, where it mainly occurs as conjugate forms with glucuronic acid or sulfate.

Urolithins A, B, C and D have all shown the ability to extend the lifespan of the nematode *Chaenorhabditis elegans* (between 19.0% and 45.4%). Urolithin A activated mitophagy throughout the duration of the treatment. Mitophagy is the process by which damaged or superfluous mitochondria are removed by autophagy. The results further suggested that urolithin A treatment activated mitochondrial biogenesis in older worms. Pharyngeal pumping rate and mobility was improved by urolithin A treatment during aging. Similar effects were observed with urolithin B, whereas urolithin C and urolithin D showed mild to no benefit (Ryu et al. 2016). Treatment with urolithin A has shown improved endurance capacity (42% greater running endurance) and increased muscle function (57% greater level of spontaneous exercise) in old mice. Lean muscle mass did not increase, suggesting that urolithin A improves muscle cell quality rather than quantity. At the molecular level, treatment with urolithin A led to a general tendency for a higher expression of autophagy and mitophagy transcripts (Ryu et al. 2016).

Furthermore, urolithin A has shown promising effects on mitochondrial health in a clinical trial. Urolithin A stimulated mitochondrial biogenesis in the skeletal muscle of humans following 28 days of oral administration at doses of 500 mg/d and 1000 mg/d (Andreux et al. 2019).

Mitochondria are the central energy producers of the cell and play a vital role in a wide range of cellular processes, including regulation of cell homeostasis. An intricate regulatory network, balancing the generation of new mitochondria (mitochondrial biogenesis) and removal of damaged mitochondria (mitophagy), forms the basis of healthy aging and longevity.

Mitophagy efficiency decline with age, leading to progressive accumulation of damaged and/or superfluous mitochondria, deterioration of cellular function, and ultimately cell death. Most aging-related diseases, particularly neurodegenerative diseases, have a mitochondrial involvement. Mitochondrial dysfunction has been associated with such diverse diseases as diabetes, cancer, cardiovascular disease, Alzheimer's Disease, Parkinson's Disease, multiple sclerosis, amyotrophic lateral sclerosis, age-related macular degeneration, chronic inflammation and autoimmune disorders (Haas 2019). The significance of impaired or defective mitophagy is well-known for the development of Alzheimer's Disease (Fang et al. 2019) and Parkinson's Disease (Srivastava 2017).

Poor mitochondrial function in the skeletal muscle has been closely linked to slow walking speed and poor muscle strength in elderly individuals. Decline in muscle function in the elderly is often part of a larger syndrome, termed frailty, which is an important risk factor for disability, hospitalization and mortality. A decrease in mitophagy-related gene expression was reported to correlate with slower walking speed in frail elderly (Drummond et al. 2014). Also, the phenomenon sarcopenia is closely linked to mitochondrial dysfunction. Sarcopenia is defined by a significant loss of skeletal muscle strength and power, muscle mass and quality of life (Ferri et al. 2020).

Emerging evidence suggests that enhanced mitophagy promotes healthy aging as well as delays the onset and progression of various age-related pathologies. Thus, mitophagy modulation may serve as a potential therapeutic approach to alleviate age-associated weaknesses.

There is thus substantial evidence suggesting that urolithins have beneficial effects in relation to delaying certain aging processes linked to mitochondrial function, and therefore urolithins might be a valuable food supplement for the aging population. However, isolated urolithins may in general not be administrated directly to humans due to pending regulatory approval procedures. Urolithin A has been GRAS approved in the US, but not authorized as novel food in EU yet (Djedjibegovic et al. 2020). Leading researchers have expressed concern about using urolithins as isolated compounds in foodstuffs, since urolithins are not natural dietary compounds (Espín et al. 2013).

An alternative to the consumption of isolated urolithins would be to use natural sources of ellagitannins such as pomegranate and certain fruits and nuts, such as raspberry, strawberry, blackberry and walnut, and rely on the *in vivo* catabolization of ellagitannins and/or ellagic acid by the gut microbiota to urolithins.

However, ingesting natural sources of ellagitannins such as pomegranate is not a generally applicable alternative, as not all individuals have the appropriate gut microbiota to produce the desired urolithin metabolites.

At least three types of urolithin producers or metabotypes have been documented in a number of clinical trials conducted by several research groups. Firstly, there are individuals who are described as not producing the final urolithin metabolites or even any kind of urolithins, or only traces thereof. These individuals are often referred to as "non-producers". According to different clinical trials, the group of non-producers accounts for 5-30% of a population (Tomas-Barberan et al. 2014; Li et al. 2015). Reports of absence of excreted urolithin by individuals in this group should however be understood as an analytical issue rather than a *de facto* proof that these individuals do not produce urolithins at all (Cerdá et al. 2004; Nuñez-Sánchez et al. 2014; Truchado et al. 2012; Tomás-Barberán et al. 2017). This was pragmatically addressed in an intervention study (Li et al. 2015) in which participants with a baseline urolithin A content in urine of up to 2.9 µg/mg creatinine were classified as non-producers. Based on the observations by Li et al. (2015), we define non-producers as individuals with a maximum amount of final urolithins in the urine of <5 µg/mg creatinine.

Secondly, a predominating group of individuals who only produce urolithin A as the final metabolite has been described (González-Sarrías et al. 2017; Tomas-Barberan et al. 2014). This group of exclusive urolithin A-producers, UA-producers, accounts for 25-80% of a population. Herein we propose a broader definition of urolithin A producers, namely individuals who produce at least twice as much urolithin A as either urolithin B or isourolithin A. The total amount of a given (final) urolithin is the sum of both the aglycone and its conjugates.

Finally, a more heterogeneous group has been defined consisting of individuals who produce urolithin A in addition to urolithin B and/or isourolithin A. This group of mixed producers is estimated to make up approx. 10-50% of a given population. Other metabotypes may exist, since the majority of the metabotype studies have been conducted in a Spanish population which may not be representative for all cultures and ethnic groups.

Herein we define the group of mixed producers as individuals who produce either urolithin B or isourolithin A in an amount corresponding to at least 50% of the urolithin A production. The total amount of a given urolithin is calculated as the sum of the amount of the aglycone and the amounts of its conjugates.

The straightforward interpretation of the occurrence of these groups is that the gut microbiome of the individuals belonging to each group is different in one or more aspects. Genetic polymorphisms or differences in the composition or functionality of the gut microbiota will contribute to the different response of individuals to the intake of ellagitannin-rich foods. In this regard, a highly variable metabolism between individuals has been described for some phenolic compounds besides ellagitannins, such as isoflavones, lignans and procyanidins (Ávila-Gálvez et al. 2020).

It remains yet to be established which gut microorganisms are responsible for the complete catabolic conversion of ellagitannins to urolithins. A number of clinical trials and *in vitro* studies have contributed to shedding light on this question, so that at least some bacteria groups, genera and species have been recognized as playing a role in the transformation. The genus *Gordonibacter,* belonging to the family Eggerthellaceae and the phylum Actinobacteria, has been linked to urolithin production in several clinical studies conducted in Spain (Gonzalez-Sarrias et al. 2017; Cortés-Martín et al. 2019; Romo-Vaquero et al. 2015).

The bacteria species *Akkermansia muciniphila* (Akkermaniaceae, Verrucomicrobia) is capable of producing ellagic acid from pomegranate ellagitannins *in vitro.* This species was found at 33-fold and 47-fold higher concentrations in stool samples of UA-producers compared to non-producers at baseline and after 4 weeks of pomegranate extract administration, respectively (Li et al. 2015).

Lactic acid bacteria and *Bifidobacteria* have been reported to decrease in mixed producers (but not UA-producers) upon intake of pomegranate extract (González-Sarrías et al. 2017). On the other hand, another clinical study reported that *Bifidobacterium* increased exclusively in mixed producers upon walnut intake (Garcia-Mantrana et al. 2019). Further, a higher abundance of *Bifidobacterium* (Bifidobacteriaceae, Actinobacteria) was found in postpartum women, who were UA-producers, than in postpartum mixed producers (Cortés-Martín et al. 2019).

Similar cases of conflicting reports on gut microbiota composition under certain conditions and in certain metabotypes seem to be more the rule than the exception. In short, the breakdown of ellagitannins to urolithins has only been partly elucidated and it is likely that more routes exist, when it comes to the involved microorganisms.

The hypothesis has been put forward, that mixed producers possess a less sound gut health compared with UA-producers. Thus, in several smaller clinical studies, mixed producers were overrepresented among patients with illnesses associated with gut dysbiosis, such as metabolic syndrome or colorectal cancer (Tomas-Barberan et al. 2014; Selma et al. 2018; Nuñez-Sánchez et al. 2014). Furthermore, it has been observed that overweight-obese individuals of the mixed producer metabotype are at higher risk of cardiovascular diseases than similar individuals who are UA-producers, when evaluating this risk from a number of cardiovascular biomarkers (González-Sarrías et al. 2017; Selma et al. 2018). In one such study it turned out that chronic consumption of an ellagitannin-rich pomegranate extract showed a significant and specific hypolipidermic effect in mixed producers (González-Sarrías et al. 2017).

Yet another study found that mixed producers prevailed among overweight-obese versus normoweight individuals (Selma et al. 2016). This study also found that Gordonibacter levels were negatively correlated with the mixed producer metabotype.

Based on the above examples from the scientific literature, it is suggested that mixed producers as defined herein have a suboptimal gut microbiota which leads to a way of metabolizing ellagitannins that further results in an overproduction of urolithin B vs. urolithin A and thus indirectly a relatively low amount of urolithin A.

### SUMMARY OF THE INVENTION

It is an objective of the present invention to enable mixed producers as defined herein, particularly men and women above 40 years of age, to benefit from the physiological advantages of urolithins in terms of healthy aging due to improved mitochondrial function and gut health. Health benefits of improved mitochondrial function include retention of muscle strength, preservation of a healthy cardiac and immune system and avoidance of neurodegenerative disorders.

An increased level of urolithin can typically be achieved *in vivo* by ingesting food sources containing ellagitannins and/or ellagic acid, which by metabolization results in the production of urolithins as discussed hereinabove. However, based on recent scientific findings, it also seems to matter which urolithins are produced, as the presence of urolithin B as a main metabolite is correlated with various metabolic disorders, in particular exemplified by the mixed producer metabotype discussed hereinabove. Human subjects having this metabotype may well increase their level of produced final urolithins by ingesting food sources containing ellagitannins and/or ellagic acid but will not thereby change their underlying metabotype. For human subjects of the mixed producer metabotype this means that increasing the level of final urolithins alone will not automatically address any metabolic disorders they may have. The mixed producer metabotype should in general be perceived as an indicator of a less optimal health of state. In terms of improving mitochondrial function, it may be at least as important to alter the mixed producer metabotype to a UA-producer metabotype than to simply raise internal urolithin levels.

It has now been found by the inventors that administering an oral composition comprising a source of ellagitannins, such as a preparation of the husk or fruit of *Punica granatum* in combination with an effective dose of one or more human milk oligosaccharides (HMOs), leads to an alteration of urolithin production in individuals who are considered "mixed urolithin producers", or just "mixed producers". As defined herein, mixed producers produce urolithin B and/or isourolithin A as final urolithin metabolites, besides urolithin A, upon ingesting food sources containing ellagitannins and/or ellagic acid. Furthermore, this oral composition also leads to a higher level of urolithin A production, since the building stones of urolithins are now lead through another metabolic pathway (Figure 1).

In our understanding urolithin A-producers (UA-producers) may also produce small amounts of urolithin B or isourolithin A, but urolithin A is by far the predominant final metabolite. Herein we thus define mixed producers as individuals with a production of either urolithin B or isourolithin A reaching at least 50% of the production of urolithin A.

The alteration of metabotype from mixed producer to UA-producer following administration of an oral composition comprising a source of ellagitannins, such as a preparation of the husk or fruit of *Punica granatum* in combination with an effective dose of one or more human milk oligosaccharides (HMOs) referred to above, may not be achieved by a corresponding amount of pomegranate preparation alone or another source of ellagitannins or ellagic acid alone.

**In a first aspect,** the present invention thus provides a composition comprising
a. a source of ellagitannins, and
b. an effective dose of one or more human milk oligosaccharides (HMOs).

The combined effect of these substances is a shift in metabotype from mixed producer to UA-producer in terms of urolithin B/urolithin A ratio or isourolithin A/urolithin A ratio.

The invention also relates to a method for the identification and treatment of mixed producers in order to make these individuals capable of benefitting from the positive health effects of improved mitochondrial function, including increased mitophagy. This is especially true for middle-aged and elderly individuals (+40). The identification is based on analyzing a urine sample obtained from an individual after said individual has ingested a standardized dose of ellagitannins for seven consecutive days.

**In a second aspect,** the present invention provides a composition according to the first aspect for use in the treatment of a human subject identified as being a mixed producer, said identification comprising the following steps:
a) Orally administering to the human subject a standardized dose of ellagitannins for seven consecutive days,
b) On the eighth day after the human subject has ingested the first standardized dose of ellagitannins, obtaining a urine sample from the human subject,
c) Analyzing the urine sample for the presence and levels of urolithin A, urolithin B and isourolithin A,
d) Establishing the levels of urolithin A, urolithin B and isourolithin A,
e) Determining whether the subject is a UA-producer, mixed producer or non-producer, based on the comparison of the subject levels with relevant reference levels,
wherein a mixed producer is defined by having a total amount of either urolithin B or isourolithin A which is at least 50% of the total amount of urolithin A in said urine sample.

**In a third aspect,** the invention also relates to a method of treatment of a human subject identified as being a mixed producer, said method comprising
a) Orally administering to the human subject a standardized dose of ellagitannins for seven consecutive days,
b) On the eighth day after the human subject has ingested the first standardized dose of ellagitannins, obtaining a urine sample from the human subject,
c) Analyzing the urine sample for the presence and levels of urolithin A, urolithin B and isourolithin A,
d) Establishing the levels of urolithin A, urolithin B and isourolithin A;
e) Determining whether the subject is a UA-producer, mixed producer or non-producer, based on the comparison of the subject levels with the relevant reference levels,
f) administering a therapeutically effective dose of a composition according to the first aspect comprising a source of ellagitannins together with one or more human milk oligosaccharides (HMOs), to any human subject identified as belonging to mixed producers; and
g) optionally monitoring said identified subject for an alteration in the relative levels of final urolithins, as measured in a urine sample, over a period of at least 30 days, such as 30 days, 60 days or preferably 90 days,
wherein a mixed producer is defined by having a total amount of either urolithin B or isourolithin A which is at least 50% of the total amount of urolithin A in said urine sample.

**In a fourth aspect,** the present invention also relates to a method for altering the metabotype in a human subject from a mixed producer to a UA-producer, wherein a mixed producer is identified by
a. Orally administering to the human subject a standardized dose of ellagitannins for seven consecutive days,
b. On the eighth day after the human subject has ingested the first standardized dose of ellagitannins, obtaining a urine sample from the human subject,
c. Analyzing the urine sample for the presence and levels of urolithin A, urolithin B and isourolithin A,
d. Establishing the levels of urolithin A, urolithin B and isourolithin A,
e. Determining whether the subject is a UA-producer, mixed producer or non-producer, based on the comparison of the subject levels with relevant reference levels,
wherein a mixed producer is defined by having a total amount of either urolithin B or isourolithin A which is at least 50% of the total amount of urolithin A in said urine sample, and wherein said method comprising administering a composition according to any one of claims 1-8 to said human subject for a period of at least 2 weeks.

**In a fifth aspect,** the present invention also relates to a method for increasing the production of urolithins, in particular urolithin A metabolites, in the human gut, as measured in plasma and/or urine, said method comprising administering a composition according to the first aspect as described above to a human subject, in particular a middle-aged or elderly (40+) human subject and in particular a human subject of the mixed producer metabotype. The composition may be administered as an ingestible medicament, as a medical device, a dietary supplement, a food additive or a medical food or a food for special medical purpose. Preferably, the one or more HMOs in the composition is at a dosage of between 500 mg and 10000 mg HMOs/day, such as at least 500 mg HMOs/day, such as at least 750 mg HMOs/day, such as at least 1000 mg HMOs/day, such as at least 2000 mg HMOs/day, such as at least 5000 mg HMOs/day.

### BRIEF DESCRIPTION OF THE FIGURES

**Figure 1****:** Gut microbiota catabolism of pomegranate ellagitannins (punicalagin) to urolithins, and differences between UA-producers (Metabotype A) and mixed producers (Metabotype B). The transient intermediate metabolite luteic acid has not yet been detected. Final urolithins are urolithin A, urolithin B and isourolithin A. All other urolithins are defined as intermediary urolithins (Garcia-Villalba et al. 2017).

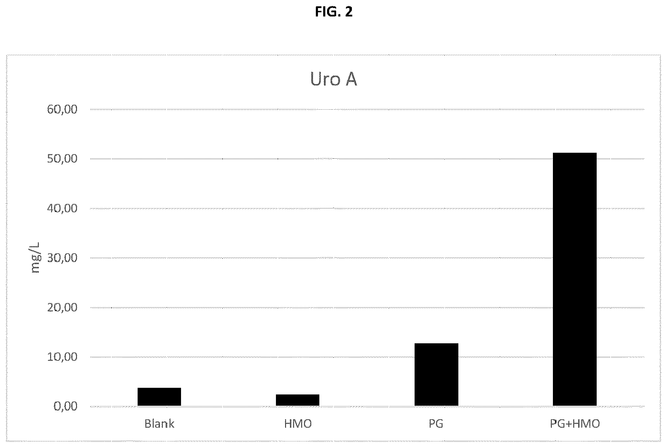

**Figure 2****:** Average production of urolithin A across 9 donors during 0-48h upon incubation of three different substrates, i.e. 3 g/L HMO, 1 g/L pomegranate extract (PG) and 3g/L HMO + 1g/L PG. A negative control (blank) was also included.

### DEFINITIONS

**Aglycone:** The noncarbohydrate group of a glycoside molecule.

**Autophagy:** Autophagy is the regulated digestion within a cell of cytoplasmic elements which it no longer requires.

**Bifidogenic:** A substance or compound that promotes the growth of bifidobacteria in the intestinal tract is said to be bifidogenic.

**Biogenesis:** The production of new living organisms or organelles.

**Catabolization:** The process in which molecules are broken down into smaller units through a set of metabolic pathways.

**Conjugate:** A compound formed by the joining of two or more chemical compounds.

**Decoy receptor:** A receptor that is able to recognize and bind specific growth factors or cytokines efficiently but is not structurally able to signal or activate the intended receptor complex.

**Ellagitannin:** The ellagitannins are a diverse class of hydrolysable tannins, a type of polyphenol formed primarily from the oxidative linkage of galloyl groups in 1,2,3,4,6-pentagalloyl glucose. Ellagitannins yield ellagic acid on hydrolysis. Examples of ellagitannins found in pomegranate fruit are: punicalagins A and B, punicalins, punicalin isomers.

**Final urolithins:** Final urolithins comprise urolithin A, urolithin B and isourolithin A. These urolithins are the measurable final metabolites of the catabolic conversion of ellagitannins and/or ellagic acid in the human gut. In plasma and urine, the urolithins are mainly found in their conjugate form.

**Fucosylated molecule:** A molecule to which one or more fucose sugar units have been added.

**Gut microbiota:** The gut microbiota is the microbial communities inhabiting the gastrointestinal tract.

**Homeostasis:** The state of steady internal, physical and chemical conditions maintained by living systems.

**Human milk oligosaccharide (HMO):** Human milk oligosaccharide, abbreviated as HMO, are complex carbohydrates found in human breast milk. The HMOs have a core structure comprising a lactose unit at the reducing end that can be elongated by one or more β-N-acetyl-lactosaminyl and/or one or more β-lacto-N-biosyl units, and which core structure can be substituted by an α-L-fucopyranosyl and/or an α-N-acetyl-neuraminyl (sialyl) moiety. In this regard, the non-acidic (or neutral) HMOs are devoid of a sialyl residue, and the acidic HMOs have at least one sialyl residue in their structure. The non-acidic (or neutral) HMOs can be fucosylated or nonfucosylated.

**Husk:** The husk is a usually dry or membranous outer covering of various seeds and fruits. The pomegranate husk is comprised of two parts: the pericarp, which provides an outer cuticle layer and fibrous mat, and the mesocarp, which is the spongy tissue and inner fruit wall where the fleshy arils are attached.

**Hydrolysis:** The chemical process in which a molecule is cleaved into two parts by the addition of a molecule of water.

**Intermediary urolithins:** Intermediary urolithins comprise urolithin M-5, urolithin M-6, urolithin M-7, urolithin C, urolithin D and urolithin E. These urolithins are the measurable intermediary metabolites of the catabolic conversion of ellagitannins and/or ellagic acid to the final metabolites urolithin A, urolithin B and isourolithin A in the human gut.

**Metabolite:** A metabolite is any substance produced during metabolism, i.e. digestion or other physiological chemical processes.

**Metabotype:** A metabolic phenotype, i.e. the kind of metabolism characterizing an individual.

**Microbiome:** The microbiome comprises all of the genetic material within a microbiota, which is the entire collection of microorganisms in a specific niche, such as the human gut.

**Mitochondria:** Mitochondria are membrane-bound cell organelles that function as power generators of the eukaryotic cell, converting oxygen and nutrients into adenosine triphosphate (ATP). Mitochondria also store calcium for cell signalling activities, generate heat and mediate cell growth and death. Mitochondria are unlike other cellular organelles in that they have two distinct membranes and a unique genome. Furthermore, they reproduce by binary fission.

**Mitophagy:** The selective degradation of dysfunctional or superfluous mitochondria by autophagy.

**Mixed producer:** An individual who produces urolithin B and/or isourolithin A in addition to urolithin A as final urolithins. In this context a mixed producer is defined as an individual who produces an amount of either urolithin B or isourolithin A corresponding to at least 50% of the urolithin A production, as measured in plasma or urine. The total amount of a given urolithin is calculated as the sum of the amount of the aglycone and the amounts of its conjugates.

**Non-producer:** An individual who does not produce any of the final metabolites urolithin A, urolithin B or isourolithin A, or only does so in low amounts (< 5 µg/mg creatinine in urine, as defined herein).

**Pericarp:** The outer layer of a ripe fruit.

**Pomace:** The solid remains of fruits after pressing for juice or oil.

**Punicoside:** The term punicosides refers to the punicalagins and punicalins in pomegranate fruit, including punicalagin A and B, punicalin A and B and punicalin isomers.

**Prebiotics:** Dietary ingredients, usually oligosaccharides, that provide a health benefit to the host mediated by the modulation of the human gut microbiota.

**Skeletal muscle:** Skeletal muscles are the most common of the three types of muscles in the body. Most skeletal muscles are attached to bones by tendons, and they produce all the movements of body parts in relation to each other. Skeletal muscles are under voluntary control of the somatic nervous system.

**UA-producer:** an individual who produces urolithin A predominantly as a final urolithin. In this context a UA-producer is defined as an individual who produces more than twice as much urolithin A than either urolithin B or isourolithin A as measured in urine or plasma. The total amount of a given urolithin is found by addition of the amount of the aglycone and the amounts of its conjugates.

### DETAILED DESCRIPTION OF THE INVENTION

It is as mentioned an objective of the present invention to enable "mixed producers" as defined herein, particularly men and women above 40 years of age, to benefit from the physiological advantages of urolithins in terms of healthy aging due to improved mitochondrial function and gut health. This is based on the findings discussed hereinabove that urolithin A stimulate mitophagy. Other urolithin metabolites, including urolithin B, presumably also have beneficial effects on cell and mitochondrial function. Health benefits of an improved mitochondrial function include retention of muscle strength, preservation of a healthy cardiac and immune system and avoidance of neurodegenerative disorders.

Mixed producers are presumably a rather heterogeneous group with various underlying causes for their relatively high production of urolithin B and/or isourolithin A. The gut microbiome of mixed producers has been described as having a lower abundance of *Bifidobacterium* (Bifidobacteriaceae, Actinobacteria) in postpartum women than in corresponding UA-producers (Cortés-Martín et al. 2019), being negatively correlated with *Gordonibacter* (Selma et al. 2016; Romo-Vaquero et al. 2015), harbouring a higher abundance of Coriobacteriaceae (a family of Actinobacteria) compared with UA-producers (Romo-Vaquero et al. 2019) and having a higher richness of bacteria compared with UA-producers (Garcia-Mantrana et al. 2019; Romo-Vaquero et al. 2019).

An increased level of urolithins in plasma can typically (i.e. except for the UM-0 metabotype described above) be achieved *in vivo* by ingesting food sources containing ellagitannins and/or ellagic acid, which by metabolization results in the production of urolithins in the gut as discussed hereinabove. However, based on recent scientific findings, it seems to matter which urolithins are produced, as the presence of urolithin B as a main metabolite is correlated with various metabolic disorders.

Several clinical studies have thus found that mixed producers are overrepresented among individuals characterized by some kind of gut dysbiosis compared with a healthy population. Mixed producers have been shown to be overrepresented among patients suffering from colorectal cancer or metabolic syndrome (Tomas-Barberan et al. 2014; Selma et al. 2018; Nuñez-Sánchez et al. 2014). Furthermore, it has been observed that overweight-obese mixed producers are at higher risk of cardiovascular diseases than overweight-obese UA-producers, based on the measurement of a number of cardiovascular biomarkers (González-Sarrías et al. 2017; Selma et al. 2018). Another study found that mixed producers prevailed among overweight-obese versus normoweight individuals (Selma et al. 2016). Thus, there is mounting evidence that being a mixed producer is a less optimal state of health than being a UA-producer, obviously when it comes to gut health, but probably also in other health aspects influenced by gut health.

These findings suggest that even if mixed producers suffering from the mentioned metabolic disorders would potentially achieve an increased total level of urolithins by ingesting food sources containing ellagitannins and/or ellagic acid, this would not in itself change their underlying metabotype and neither would it automatically address their metabolic disorders.

We suggest that urolithin B as a main human metabolite is an indicator of a suboptimal gut microbiota or some degree of gut dysfunction, even if urolithin B as an isolated compound may possess some of the beneficial properties of urolithin A. In other words, we do not consider urolithin B as such to constitute a potential health problem, rather the gut microbiomic conditions leading to its overproduction. The mixed producer metabotype presents such microbiomic conditions.

We therefore find it reasonable to expect that mixed producers would benefit from changing their metabotype towards a UA-producer and thereby achieve an improved gut health and eventually also the positive effects on mitophagy associated with a higher level of urolithin A.

Only a few studies deal with conversions between metabotypes. The metabotype of healthy, lactating women was determined four times during the first year after having given birth (Cortés-Martín et al. 2019). It turned out that 20% of the women from the mixed producer group changed metabotype to become UA-producers for unknown reasons. Another clinical study reports the conversion of non-producers to urolithin producers among healthy overweight-obese individuals. Conversions in three out of six non-producers were observed upon intake of pomegranate extract. The circumstances of these conversions seem somewhat unclear, since the group of converted non-producers differed at baseline from obligate non-producers in their significantly higher level of *Gordonibacter.* Furthermore, the study did not report the actual urolithin production for the converted non-producers (González-Sarrías et al. 2017).

In other words, conversion of urolithin metabotypes seems to be possible in some cases, but until now it has not been known how to manage such a conversion in a controlled way (Cortes-Martin et al. 2018).

The inventors have now found that by administering a composition which comprises a source of ellagitannins, such as a preparation of the fruit of *Punica granatum,* e.g. a pomace extract, and one or more human milk oligosaccharides (HMOs), individuals identified as mixed producers may change their metabotype to UA-producer, and simultaneously increase their production of urolithin A metabolites.

This was demonstrated both *in vitro* and *in vivo.* **Example 3** herein describes a study designed to assess the impact *in vitro* of ¹⁾ an HMO blend, ²⁾ a pomegranate (PG) extract as an example of a source of ellagitannins, and ³⁾ the combination of both (PG+HMO) on the profile of urolithin metabolism, i.e. urolithin metabotype, by the gut microbiota of nine randomly selected human donors in a short-term *in vitro* colonic simulation model.

The study demonstrated (see Example 3, Table 1) that after incubating the fecal samples from the nine human donors with PG extract, three donors (donors 2, 3 and 6) proved to be mixed producers. After PG+HMO treatment donor 2 and 3 changed their metabotype to pronounced urolithin A producers. The percentage of produced isourolithin A compared with urolithin A changed from 351% to 0% for donor 2 and 936% to 4 % for donor 3. Furthermore, the production of urolithin A increased with a factor 3,5*10³ and 1,8*10⁴, respectively, for the two donors. For donor 6, a more modest change was seen from 149% to 106%, thus donor 6 remained a mixed producer. For the urolithin A producers, a less clear picture was seen. In several donors the percentage of isourolithin A compared with urolithin A increased upon PG+HMO treatment.

These findings strongly indicate that the addition of HMO to pomegranate extract elicits or entails a metabotype shift from mixed producer towards UA-producer. The decrease in isourolithin A/urolithin A ratio is of surprising magnitude. In two out of three mixed producers a shift from clear mixed producers to almost exclusive UA-producers are observed.

The combination of HMO+PG treatment in Example 3 further improved urolithin production in a synergistic manner in fecal sample incubations originating from 7 out of 9 donors (i.e. all donors but donors 4 and 9). The effect was most pronounced for the production of urolithin A, which increased by >300% across the 9 donors during the study (see Figure 2).

The findings of the *in vitro* study discussed in Example 3 were corroborated *in vivo.* **Example 4** herein describes a study where 9 separate cases were followed with the main purpose to investigate whether the metabotype formed by the daily consumption of pomegranate extract was changed by the complementary addition of an HMO blend to pomegranate extract. Nine participants were thus followed, i.e. 6 men and 3 women between the ages of 26 - 91, some of which had already been taking pomegranate extract (1 g/day) continuously for several months or years, either as a sole active ingredient in tablet form or combined with other dietary supplement ingredients in tablet form. The nine participants had no connection with the donors in Example 3.

The participants initially went through 3 weeks of daily consumption of pomegranate extract (PG treatment), followed by the collection of a sample of morning urine. Then all participants went through 3 weeks of daily consumption of pomegranate extract + HMO (PG+HMO treatment), followed by the collection of a sample of morning urine.

The metabotype of the participants was established both after PG treatment and after PG+HMO treatment and compared (Example 4, Table 2). After the initial 3 weeks of PG treatment, 6 of the participants were categorized as UA-producers while 3 were categorized as mixed producers.

Of the mixed producers, two participants were converted to UA-producers during the following 3 weeks of PG+HMO treatment. The most pronounced change was seen in a mixed producer, whose ratio between urolithin B compounds and urolithin A compounds shifted from 263% to 13%. In the same mixed producer, the production of urolithin A metabolites increased with 350% from PG treatment to PG+HMO treatment. Among UA-producers no major changes were observed. Three UA-producers remained at approximately the same urolithin B/urolithin A ratio, one obtained a lower ratio, while two obtained a higher ratio.

These findings *in vivo* corroborate the conclusions of Example 3 and again strongly indicate that the addition of HMO to pomegranate extract elicits or entails a metabotype shift from mixed producer towards UA-producer. In two out of three mixed producers in Example 4 a shift from mixed producer status to UA-producer is observed. Furthermore, data indicates that an increase in urolithin A metabolites may take place simultaneous with the metabotype shift from mixed producer to UA-producer.

**In a first aspect,** the present invention thus provides a composition comprising
a. a source of ellagitannins, and
b. one or more human milk oligosaccharides (HMOs).

Following the administration of the composition according to the first aspect to a human subject having been identified as a mixed producer as defined herein, said human subject may change metabotype to UA-producer, defined by having a total amount of urolithin A which is at least twice as much as the total amounts of either urolithin B or isourolithin A in a urine sample collected upon treatment. The total amount of a given urolithin is calculated as the sum of the amount of the aglycone and the amounts of its conjugates.

The source of ellagitannins may be an extract of the fruit of *Punica granatum,* obtainable by methods known in the art. In some embodiments, the extract of *Punica granatum* is obtainable from the husk or pericarp of *Punica granatum.* In a preferred embodiment, the present invention thus provides a composition comprising
a. an extract of the husk of *Punica granatum,* and
b. one or more human milk oligosaccharides (HMOs).

The source of ellagitannins may also be an extract of the fruit of *Punica granatum* obtained from the pomace of *Punica granatum,* i.e. by extracting the remains of squeezed fruits. The extract is preferably a dry extract, such as a powder or a granulate. Other methods of preparing an extract of the husk or fruit of *Punica granatum* well known in the art, are also within the scope of the invention.

Specific embodiments relate to extracts of the husk or fruit of *Punica granatum* comprising at least 40% polyphenols. The extract of the fruit of *Punica granatum* according to the present invention preferably further comprises at least 30% punicosides and/or at least 20% punicalagins.

The source of ellagitannins may also be the seeds of *Juglans regia,* dried or as an extract obtainable by methods known in the art. Another source of ellagitannins is a preparation of the fruit of *Vitis rotundifolia.* The preparation may be an extract of the fruit of *Vitis rotundifolia* obtained from the pomace of *Vitis rotundifolia,* i.e. by extracting the remains of the squeezed fruits.

In an embodiment, the HMO is selected from neutral HMOs or acidic HMOs. The neutral HMO can be one or more fucosylated HMOs or one or more non-fucosylated HMOs.

The composition according to the first aspect of the present invention may be such that the ratio (w/w) between the source of the ellagitannins and the one or more human milk oligosaccharides (HMOs) is comprised in the range of 5:1-1:20.

In one embodiment, the composition according to the first aspect comprises an extract of the husk of *Punica granatum,* combined with one or more HMOs selected from neutral HMOs or acidic HMOs. The neutral HMO can be one or more fucosylated HMOs or one or more non-fucosylated HMOs.

**In a second aspect,** the present invention provides a composition according to the first aspect for use in the treatment of a human subject identified as being a mixed producer, said identification comprising the following steps:
a) Orally administering to the human subject a standardized dose of ellagitannins for seven consecutive days,
b) On the eighth day after the human subject has ingested the first standardized dose of ellagitannins, obtaining a urine sample from the human subject,
c) Analyzing the urine sample for the presence and levels of urolithin A, urolithin B and isourolithin A,
d) Establishing the levels of urolithin A, urolithin B and isourolithin A,
e) Determining whether the subject is a UA-producer, mixed producer or non-producer, based on the comparison of the subject levels with relevant reference levels,
wherein a mixed producer is defined by having a total amount of either urolithin B or isourolithin A which is at least 50% of the total amount of urolithin A in said urine sample.

**In a third aspect,** the invention also relates to a method of treatment of a human subject identified as being a mixed producer, said method comprising:
a) Orally administering to the human subject a standardized dose of ellagitannins for seven consecutive days,
b) On the eighth day after the human subject has ingested the first standardized dose of ellagitannins, obtaining a urine sample from the human subject,
c) Analyzing the urine sample for the presence and levels of urolithin A, urolithin B and isourolithin A,
d) Comparing the level of urolithin A, urolithin B and isourolithin A with metabotype reference levels for UA-producers and mixed producers;
e) Determining whether the subject is a UA-producer, mixed producer or non-producer, based on the comparison of the subject levels with the reference levels,
f) administering a therapeutically effective dose of a composition according to the first aspect comprising a source of ellagitannins together with one or more human milk oligosaccharides (HMOs), to any human subject identified as being a mixed producer; and
g) optionally monitoring said identified subject for the production of one or more final urolithins, as measured in a urine sample, over a period of at least 30 days, such as 30 days, 60 days or preferably 90 days,
wherein a mixed producer is defined by having a total amount of either urolithin B or isourolithin A which is at least 50% of the total amount of urolithin A in said urine sample.

**In a fourth aspect,** the present invention also relates to a method for altering the metabotype in a human subject from a mixed producer to a UA-producer, wherein a mixed producer is identified by
a. Orally administering to the human subject a standardized dose of ellagitannins for seven consecutive days,
b. On the eighth day after the human subject has ingested the first standardized dose of ellagitannins, obtaining a urine sample from the human subject,
c. Analyzing the urine sample for the presence and levels of urolithin A, urolithin B and isourolithin A,
d. Establishing the levels of urolithin A, urolithin B and isourolithin A,
e. Determining whether the subject is a UA-producer, mixed producer or non-producer, based on the comparison of the subject levels with relevant reference levels,
wherein a mixed producer is defined by having a total amount of either urolithin B or isourolithin A which is at least 50% of the total amount of urolithin A in said urine sample, and wherein said method comprising administering a composition according to any one of claims 1-8 to said human subject for a period of at least 2 weeks.

In a preferred embodiment, the treatment of individuals identified as mixed producers involves administering a daily dose of the composition according to the first aspect containing between 500 - 10000 mg HMOs/day and 500 - 5000 mg of an extract of the fruit of *Punica granatum*/day*.*

Human milk oligosaccharides (HMOs) are a group of complex sugars, or oligosaccharides, that are present at high concentrations in human milk. HMOs serve as metabolic substrates for select gut microbes, thus contributing to the development of the infant gut microbiota. In other words, HMOs function as prebiotic substances.

HMOs have been found to shape the infant's gut microbiota by selectively stimulating the growth of specific bacteria, especially bifidobacteria. These bifidogenic effects of HMOs are structure-specific and may vary depending on the HMO composition in the milk of different individuals.

The structural diversity represented by HMOs can be broadly divided into fucosylated (neutral HMOs), sialyated (acidic HMOs) and non-fucosylated neutral structures. Approximately 200 different HMOs have been identified so far. The building blocks of human milk oligosaccharides are the five monosaccharides D-glucose, D-galactose, N-acetylglucosamine, L-Fucose, and sialic acid. Lactose forms the reducing end of milk oligosaccharides. The amount and composition of HMOs produced in human milk are highly variable between women.

Apart from having prebiotic effects, HMOs also act as soluble decoy receptors that block the attachment of viral, bacterial or protozoan pathogens to epithelial cell surface sugars. This function as an anti-adhesive and may help prevent infectious diseases in the gut and also in the respiratory and urinary tracts.

Supplementation of 2'-fucosyllactose (2'FL) and lacto-*N*-neotetraose (LNnT) at daily doses up to 20 g to healthy adults modified the gut microbiota with the primary impact being substantial increases in relative abundance of Actinobacteria and *Bifidobacterium* in particular and a reduction in relative abundance of Firmicutes and Proteobacteria. This modulation occurred within 1- 2 weeks (Elison et al. 2016).

In another embodiment, the composition according to the first aspect comprises one or more HMOs selected from 2-fucosyllactose (2'-FL), 3-fucosyllactose (3-FL), difucosyllactose (DFL), lacto-N-tetraose (LNT), lacto-N-neotetraose (LNnT), 3-sialyllactose (3'-SL), 6-sialyllactose (6'-SL), lacto-N-fucopentaose 1 (LNFP-1) or a mixture thereof.

Preferably, the one or more HMOs comprises, consists of or essentially consists of 2'-FL and at least one of LNnT and LNT; at least one of 2'-FL and DFL and at least one of LNnT and LNT (e.g. 2'-FL, DFL and at least one of LNnT and LNT); 2'FL and 6'-SL; DFL and 6'-SL; 2'-FL, DFL and 6'-SL; 2'-FL, 6'-SL and at least one of LNnT and LNT; or 2'-FL, DFL, 6'-SL and at least one of LNnT and LNT.

In some embodiments the composition according to the first aspect is administered to a human subject who is a mixed producer as discussed hereinabove. In some embodiments, administration of a composition according to the first aspect of the invention to a human subject of the mixed producer metabotype results over time in an alteration of the gut microbiota of said human subject, which is capable of improved and/or increased catabolization of ellagitannins and/or ellagic acid into one or more of the final urolithins, especially urolithin A.

Specific embodiments relate to the use of a composition as disclosed herein for changing metabotype from mixed producer to UA-producer by administration to a human subject for a duration of at least 30 days, such as at least 50 days, such as at least 100 days, such as at least 150 days. Preferably, the composition is administered at least until the subject has obtained the profile of a UA-producer.

The present composition may be formulated in a formulation, preferably an ingestible formulation, such as a medicament, a dietary supplement, a food additive or a medical food or a food for special medical purpose. The formulation may comprise other ingredients, such as, but not limited to, an excipient, a coating agent, a flavouring agent, one or more herbal extracts or preparations, one or more probiotics, one or more nutrients, such as one or more vitamins and/or minerals. The composition may be formulated as a tablet, a pill, a capsule, a powder or a granulate. Other formulations suited for the purpose will be obvious to the skilled man and are also envisioned. The composition may be formulated as distinct formulations, wherein the first device comprises the source of ellagitannins and the second device comprises the one or more HMOs. Alternatively, the composition may be formulated as a single formulation.

By analysing the level of urolithins produced by human gut microbiota, the inventors have found a synergistic effect of an HMO blend + a preparation of the husk or fruit of *Punica granatum* compared with administering an HMO blend or a preparation of the husk or fruit of *Punica granatum* individually regarding the production of final urolithins, urolithin A and iso-urolithin A, and the intermediary urolithin, urolithin C (see Example 3).

**In a fifth aspect,** the present invention also relates to a method for increasing the production of urolithins, in particular urolithin A metabolites, in the human gut, as measured in plasma and/or urine, said method comprising administering a composition according to the first aspect as described above to a human subject, in particular a middle-aged or elderly (40+) human subject and in particular a human subject of the mixed producer metabotype. The composition may be administered as an ingestible medicament, as a medical device, a dietary supplement, a food additive or a medical food or a food for special medical purpose. Preferably, the one or more HMOs in the composition is at a dosage of between 500 mg and 10000 mg HMOs/day, such as at least 500 mg HMOs/day, such as at least 750 mg HMOs/day, such as at least 1000 mg HMOs/day, such as at least 2000 mg HMOs/day, such as at least 5000 mg HMOs/day.

In another embodiment, the method for modulating the metabotype from mixed producer to UA producer in a human subject according to the fourth aspect comprises administering a composition according to the first aspect which contains at least 75 mg ellagitannins/day, such as between 75 mg/day and 1500 mg/day, such as between 100 mg/day and 1000 mg/day, preferably at least 300 mg/day.

The preparation of the fruit of *Punica granatum* comprises preferably at least 40% polyphenols, at least 30% punicosides and at least 20% punicalagins. In some embodiments, the composition is administered to a human subject at a dosage of at least 75 mg punicalagins/day, such as between 75 mg/day and 1500 mg/day, such as between 100 mg/day and 1000 mg/day, preferably at least 300 mg/day. In some embodiments, the dosage of punicosides is at least 100 mg/day, such as between 100 mg/day and 2000 mg/day, such as between 100 mg/day and 1200 mg/day, preferably at least 400 mg/day. The dosage of polyphenols is at least 125 mg/day, such as between 125 mg/day and 2500 mg/day, such as between 250 mg/day and 1500 mg/day, preferably at least 500 mg/day.

In a preferred embodiment the composition of the present invention may be formulated as a kit comprising ¹⁾ tablets containing the source of ellagitannins, ²⁾ a powder or granulate combination of the two HMOs 2'-Fucosyllactose (2'-FL) and Lacto-N-neotetraose (LNnT) and ³⁾ instructions for use.

In another embodiment the composition of the present invention may be formulated as tablets or capsules comprising a mixture of a source of ellagitannins, 2'-Fucosyllactose (2'-FL) and Lacto-N-neotetraose (LNnT).

In another embodiment the composition of the present invention may be formulated as a kit comprising ¹⁾ tablets containing pomegranate extract, ²⁾ a powder or granulate of one or more HMOs selected from 2'-FL, 3-FL, DFL, LNT, LNnT, 3'-SL, 6'-SL, LNFP-1 or a mixture thereof, and³) instructions for use.

In another embodiment the composition of the present invention may be formulated as tablets comprising a mixture of pomegranate extract and one or more HMOs selected from 2'-FL, 3-FL, DFL, LNT, LNnT, 3'-SL, 6'-SL, LNFP-1 or a mixture thereof.

In other embodiments of the present invention, the pomegranate extract may be replaced by other natural sources of ellagitannins and/or ellagic acid, such as preparations of walnut, muscadine grape, raspberry, strawberry and/or blackberry.

### EXAMPLES

### Example 1

### Formulation of a kit comprising a pomegranate extract and a combination of HMOs

A kit consisting of a pomegranate extract and a combination of the two HMOs 2'-Fucosyllactose (2'-FL) and Lacto-N-neotetraose (LNnT) was developed. The kit consisted of a tablet formulation and a powder formulation.

A dry ethanolic extract of *P. granatum* was used for the tablet formulation. The ethanolic extract was based on pomegranate fruit pomace (Monteloeder S.L., Elche, Alicante, Spain). The content of punicalagins was min. 30%. The daily dose of the pomegranate extract was 1000 mg/4 tablets.

The following tablet excipients were added to the formulation: microcrystalline cellulose, cross-linked sodium carboxymethyl cellulose, sodium carboxymethyl cellulose, silicon dioxide, magnesium salts of vegetable fatty acids, hydroxypropyl methyl cellulose, polyethylene glycol.

As the other active ingredient, a blend of the two HMOs 2'-Fucosyllactose and Lacto-N-neotetraose (Glycom A/S, Hørsholm, Denmark) was used for a powder formulation. The mass ratio between the two HMOs was 4:1 (2'-FL/LNnT). The daily dose of the 2'-FL/LNnT-blend was 5000 mg/stick pack. The following excipient was added to the formulation: magnesium stearate (0.25 mass %).

### Example 2

### Identification of mixed producers by urine sample analysis

Mixed producers are characterized by a relatively high urolithin B and/or iso-urolithin A production compared with urolithin A production. This is possibly indicative of a suboptimal gut microbiota or even gut dysfunction. Mixed producers are identified by the following method:
A daily dose of pomegranate extract (1 g/4 tablets) containing 30% punicalagins is consumed by the individual for seven consecutive days. In the morning of the eighth day, a urine sample is collected and the urolithin content is analyzed by LC-MS/MS analysis. The individual amounts of urolithin B, isourolithin A and urolithin A in the urine sample are established, which allows the classification of the individual according to capacity to metabolize ellagitannins into final urolithins. The total amount of a given urolithin is the sum of both the aglycone and its conjugates. A mixed producer is defined by having a total amount of either urolithin B or isourolithin A which is at least 50% of the total amount of urolithin A in said urine sample.

### Example 3

### Modulation of urolithin metabotype in vitro with a combination of an ellagitannin-rich food source and an HMO blend

The following study was designed to assess the impact of an HMO blend, a pomegranate extract (PG) and the combination of both on the profile of urolithin metabolism, i.e. urolithin metabotype, by the gut microbiota of nine randomly selected human donors in a short-term *in vitro* simulation. The company ProDigest's (https://www.prodigest.eu/en) short-term colonic simulation model was used to study the interaction between test products and the gut microbiome.

All donors were between 50 and 70 years old, healthy and with no history of antibiotic use 6 months before the experiment. Fecal material was collected, and fecal suspensions prepared and mixed with a cryoprotectant. The obtained suspensions were aliquoted, flash frozen and then preserved at -80°C. Just before the experiment, fecal samples were defrosted and immediately added to the reactors.

The reactors were incubated for 48 hours (37°C, shaken at 90 rpm, anaerobic conditions) with a sugar-depleted nutritional medium containing basal nutrients of the colon. Samples from the reactors were collected at 0h (baseline), after 24 h and after 48h (follow-up). Each fecal sample was used for 4 different treatments:
- Control treatment (blank): only nutritional medium was added to the reactor.
- Human milk oligosaccharide (HMO) treatment: 3 g/l of an HMO blend was added to the reactor at baseline.
- Pomegranate extract (PG) treatment: 1 g/l was added to the reactor at baseline.
- Pomegranate extract + HMO (PG + HMO) treatment: 1g/l of PG and 3 g/l of an HMO blend was added to the reactor at baseline.

Incubations were performed in single repetition, resulting in 36 independent incubations.

The pomegranate extract was an ethanolic extract based on pomegranate fruit pomace (Monteloeder S.L., Elche, Alicante, Spain). The content of punicalagins was approx. 40%.

The HMO blend was a powder consisting of 2'-fucosyllactose (2'FL) and lacto-N-neotetraose (LNnT) in the mass ratio 4:1 (2'FL:LNnT). The HMO blend was provided by Glycom A/S, Hørsholm, Denmark.

The concentration of the final urolithins urolithin A, isourolithin A and urolithin B was measured at 0h, 24h and 48h. Furthermore, the concentration of the intermediary urolithins urolithin C and urolithin D was measured at 0h, 24h and 48h.

The metabotype of the donors were compared after PG treatment vs. after PG+HMO treatment (table 1). The isourolithin A concentrations given are measurements at 48h minus the concentration measured at 0h. No isourolithin production could be measured at 24h. For urolithin A, the highest of the concentrations at either 24h-0h or 48h-0h is used, since in several cases it seemed like the reactor medium environment had somehow crashed at 48h with a dramatical decrease in urolithin A as a consequence. No urolithin B was measured in any of the reactors, which can be explained by the late synthesis of this compound in the metabolic pathway of final urolithins (Figure 1). Since the precursor isourolithin A was not detected in the samples before at 48h, it is not surprising that the production of urolithin B was not initiated within the same 48 hours. In table 1, mixed producers are defined as donors where the amount of measured isourolithin A amounts to at least 50% of the amount of measured urolithin A.

**Table 1. Comparison of the metabotype of 9 donors after PG treatments vs. PG+HMO treatment in fecal sample incubations. Concentrations of isourolithin A (Iso-A) and urolithin A (Uro-A) in the nutritional medium are shown (mg/l). Isourolithin A concentrations measured at 48h (minus the concentration at 0h) are given. For urolithin A, the highest concentration at either 24h-0h or 48h-0h are given. The ratio between isourolithin A and urolithin A production is shown as the percentage of isourolithin A of urolithin A. A ratio of less than 50% renders the donor a urolithin A producer (UA). A ratio of 50% or higher renders the donor a mixed producer (mixed).**

| | **After PG treatment** | | | | **After PG+HMO treatment** | | | |
|---|---|---|---|---|---|---|---|---|
| **Donor** | **Iso-A mg/L** | **Uro-A mg/L** | **Iso-A/ Uro-A ratio** | **Metabotype** | **Iso-A mg/L** | **Uro-A mg/L** | **Iso-A/Uro-A ratio** | **Metabotype** |
| 1 | 0,00 | 1,22 | 0% | UA | 5,97 | 12,00 | 49,8% | UA |
| 2 | 1,46 | 0,41 | 351% | Mixed | 0,00 | 14,55 | 0% | UA |
| 3 | 0,88 | 0,09 | 936% | Mixed | 0,70 | 16,33 | 4% | UA |
| 4 | 0,00 | 3,99 | 0% | UA | 0,00 | 1,23 | 0% | UA |
| 5 | 0,05 | 3,78 | 1% | UA | 3,04 | 0,17 | 1812% | Mixed |
| 6 | 2,35 | 1,57 | 149% | Mixed | 3,59 | 3,39 | 106% | Mixed |
| 7 | 0,10 | 0,24 | 42% | UA | 0,17 | 0,17 | 103% | Mixed |
| 8 | 0,47 | 1,29 | 36% | UA | 0,97 | 3,06 | 32% | UA |
| 9 | 0,00 | 0,10 | 0% | UA | 0,10 | 0,36 | 27% | UA |

Three donors (donors 2, 3 and 6) proved to be mixed producer after PG treatment. After PG+HMO treatment donor 2 and 3 changed their metabotype to pronounced UA-producers. The percentage of produced isourolithin A compared with urolithin A changed from 351% to 0% for donor 2 and 936% to 4 % for donor 3. For donor 6, a more modest change was seen from 149% to 106%, thus still being a mixed producer.

For the UA-producers a less clear picture was seen. In several donors the percentage of isourolithin A compared with urolithin A increased upon PG+HMO treatment. One donor (donor 5) changed metabotype from UA producer to mixed producer. Donors 7 and 9 could be regarded non-producers due to the low measured levels of final urolithins for all samples.

Our data strongly indicates that the addition of HMO to pomegranate extract elicits a metabotype shift in mixed producers towards UA-producers. The decrease in isourolithin A/urolithin A ratio is of surprising magnitude. In 2 out of 3 mixed producers a shift from clear mixed producers to almost exclusive urolithin A producers are observed.

The *in vitro* study also presented a number of other interesting results regarding the synergistic effect of HMO+PG treatment compared with HMO treatment or PG treatment, respectively (data not shown):
A synergistic effect of HMO+PG treatment compared with HMO treatment or PG treatment, respectively, was seen for urolithin A production in donors 1, 2 and 3 and to a lesser extent in donors 6 and 8.

A synergistic effect of HMO+PG treatment compared with HMO treatment or PG treatment, respectively, was seen for isourolithin A production in donors 1 and 5 and to a lesser extent in donors 6 and 8.

A synergistic effect of HMO+PG treatment compared with HMO treatment or PG treatment, respectively, was seen for urolithin C production in donors 5 and 7 and to a lesser extent in donor 1.

Synergistic effects of HMO+PG treatment was not observed for urolithin D production.

Thus, overall, the combination of HMO+PG treatment improved urolithin production in a synergistic manner in fecal sample incubations originating from 7 out of 9 randomly assigned donors (i.e. all donors but donors 4 and 9). The effect was most pronounced for the production of urolithin A. The average data for urolithin A production across the 9 donors are presented in Figure 2. The most pronounced increases in urolithin A production were measured for the mixed producers, donor 2 and 3, where urolithin A increased with a factor 3,5*10³ and 1,8*10⁴, respectively.

### Example 4

### Treatment of mixed producers and UA-producers with a combination of an ellagitannin-rich food source and an HMO blend

A number of separate cases were followed with the purpose to investigate whether the metabotype formed by the daily consumption of pomegranate extract was changed by the complementary addition of an HMO blend to pomegranate extract. Nine participants were followed, i.e. 6 men and 3 women between the ages of 26 - 91. Some of the participants had already been taking pomegranate extract (1 g/day) continuously for several months or years, either as a sole active ingredient in tablet form or combined with other dietary supplement ingredients in tablet form. The 9 participants were all different from the donors in Example 3.

All participants initially went through 3 weeks of daily consumption of pomegranate extract (PG), followed by the collection of a sample of morning urine. Then all participants went through 3 weeks of daily consumption of pomegranate extract + HMO (PG+HMO), followed by the collection of a sample of morning urine.

Participants were instructed not to consume other ellagitannin-rich foods or beverages such as berries, nuts, pomegranates and oak aged wine the last 5 days before collection of a urine sample.

A dry ethanolic extract of *P. granatum* was used for a tablet formulation. The ethanolic extract was based on pomegranate fruit pomace (Monteloeder S.L., Elche, Alicante, Spain). The content of punicalagins was 40%. The daily dose of pomegranate extract was 1000 mg in tablet form.

A blend of the two HMOs 2'-Fucosyllactose and Lacto-N-neotetraose (2'-FL and LNnT) in the mass ratio 4:1 was administered as a powder. The daily dose of the blend was 5000 mg.

Urine samples were frozen immediately upon collection and the urolithin content was later analyzed by UPLC-MS analysis. The following urolithins were analyzed: urolithin A, urolithin B, urolithin C, urolithin D, urolithin A glucuronide, urolithin A sulfate, urolithin B glucuronide and urolithin C glucuronide. Urolithin A glucuronide was measured in the positive ionization mode, while the other compounds were measured in the negative ionization mode. The amounts of isourolithin A metabolites were not analyzed, however from the chromatograms the amounts of these compounds seemed negligible. Data were pre-processed using mzMine. Data were normalized to the urinary creatinine concentration as well as to the total intensity (all measured metabolites).

The study participants were categorized as UA-producers or mixed producers. Mixed producers are defined as individuals where the amount of measured urolithin B amounts to at least 50% of the amount of measured urolithin A. Individuals with a relatively higher urolithin A production are defined as UA-producers. No non-producers were found among the study participants.

The metabotype of the participants was compared after PG treatment vs. after PG+HMO treatment (table 2). The amount of urolithin B (calculated as the sum of the concentrations of urolithin B + urolithin B glucuronide) was compared with the amount of urolithin A (calculated as the sum of the concentrations of urolithin A + urolithin A glucuronide + urolithin A sulfate) in morning urine samples collected after PG treatment and PG+HMO treatment, respectively. Data normalized to total intensity was used. (The same conclusions were reached by using data normalized to urinary creatinine concentrations.)

**Table 2. Identification of the metabotype of 9 participants after PG treatments vs. PG+HMO treatment by comparing the relative mass-spectrometrical (MS) intensities of urolithin B compounds (Uro-B) vs. urolithin A compounds (Uro-A) in urine samples after the two treatments. Data are normalized to total MS-intensity (all measured metabolites). The ratio between urolithin B and urolithin A production is shown as the percentage of urolithin B of urolithin A. A ratio of less than 50% renders the participant a urolithin A producer (Urolithin A). A ratio of 50% or higher renders the participant a mixed producer (mixed).**

| | **After PG treatment** | | | | **After PG+HMO treatment** | | | |
|---|---|---|---|---|---|---|---|---|
| **Participant** | **Uro-B** | **Uro-A** | **Uro-B / Uro-A** | **Metabotype** | **Uro-B** | **Uro-A** | **Uro-B/ Uro-A** | **Metabotype** |
| A | 0,00050 | 0,00605 | 8% | UA | 0,00127 | 0,00327 | 39% | UA |
| B | 0,00419 | 0,00159 | 263% | Mixed | 0,00020 | 0,00151 | 13% | UA |
| C | 0,00062 | 0,01366 | 5% | UA | 0,00078 | 0,01528 | 5% | UA |
| D | 0,00041 | 0,00651 | 6% | UA | 0,00036 | 0,01117 | 3% | UA |
| E | 0,00145 | 0,00195 | 75% | Mixed | 0,00092 | 0,00682 | 13% | UA |
| F | 0,00077 | 0,01193 | 6% | UA | 0,00057 | 0,00848 | 7% | UA |
| G | 0,00066 | 0,00309 | 21% | UA | 0,00038 | 0,00396 | 10% | UA |
| H | 0,00113 | 0,00088 | 128% | Mixed | 0,00233 | 0,00077 | 304% | Mixed |
| I | 0,00017 | 0,00716 | 2% | UA | 0,00128 | 0,00871 | 15% | UA |

After PG treatment, 6 of the participants were categorized as UA producers (A, C, D, F, G, I), while 3 were categorized as mixed producers (B, E, H). Of the mixed producers, 2 participants (B, E) were converted to UA producers during the 3 weeks of PG+HMO treatment. The most pronounced change was seen in a mixed producer, whose ratio between urolithin B compounds and urolithin A compounds shifted from 263% to 13%.

Among urolithin A producers no major changes were observed. Three urolithin A producers remained at approximately the same urolithin B/urolithin A ratio, another one obtained a lower ratio, while two obtained a higher ratio. In general, the great interindividual differences made it difficult to obtain significant P-values across the data set. The amount of the urolithin A aglycone in the urine samples was however significantly higher after PG+HMO treatment than after PG treatment (P=0.047, data normalized to total intensity). This is an interesting result, since it is known from the literature that the amount of the urolithin A aglycone in urine is positively correlated with the amounts of other urolithin A metabolites, including urolithin A glucuronide (Romo-Vaquero et al. 2015). A significant increase in the total amount of urolithin A in urine upon PG+HMO treatment would be expected in a study only involving mixed producers. In this study, a pronounced increased production of urolithin A compounds was only seen in mixed producer E (350%).

Thus, the hypothesis that treatment with a combination of pomegranate extract plus HMO is capable of altering the metabotype of mixed producers to urolithin A producers, was substantiated. Furthermore, data is indicative of a beneficial effect on total urolithin A production in mixed producers.

No side effects were reported during the study.

### References

Andreux PA, Blanco-Bose W, Ryu D, Burdet F, Ibberson M, Aebischer P, Auwerx J, Singh A, Rinsch C (2019) The mitophagy activator urolithin A is safe and induces a molecular signature of improved mitochondrial and cellular health in humans. Nature Metabolism 1 (6):595-603. doi:10.1038/s42255-019-0073-4
Ávila-Gálvez MÁ, Giménez-Bastida JA, Espín JC, González-Sarrías A (2020) Dietary Phenolics against Breast Cancer. A Critical Evidence-Based Review and Future Perspectives. International journal of molecular sciences 21 (16):5718
Cerdá B, Espín JC, Parra S, Martinez P, Tomás-Barberán FA (2004) The potent in vitro antioxidant ellagitannins from pomegranate juice are metabolised into bioavailable but poor antioxidant hydroxy-6H-dibenzopyran-6-one derivatives by the colonic microflora of healthy humans. European journal of nutrition 43 (4):205-220
Cortes-Martin A, Garcia-Villalba R, Gonzalez-Sarrias A, Romo-Vaquero M, Loria-Kohen V, Ramirez-de-Molina A, Tomas-Barberan F, Selma M, Espin J (2018) The gut microbiota urolithin metabotypes revisited: the human metabolism of ellagic acid is mainly determined by aging. Food & function 9 (8):4100-4106
Cortés-Martín A, Romo-Vaquero M, Garcia-Mantrana I, Rodriguez-Varela A, Collado MC, Espín JC, Selma MV (2019) Urolithin metabotypes can anticipate the different restoration of the gut microbiota and anthropometric profiles during the first year postpartum. Nutrients 11 (9):2079
Djedjibegovic J, Marjanovic A, Panieri E, Saso L (2020) Ellagic Acid-Derived Urolithins as Modulators of Oxidative Stress. Oxidative Medicine and Cellular Longevity 2020
Drummond MJ, Addison O, Brunker L, Hopkins PN, McClain DA, LaStayo PC, Marcus RL (2014) Downregulation of E3 ubiquitin ligases and mitophagy-related genes in skeletal muscle of physically inactive, frail older women: a cross-sectional comparison. Journals of Gerontology Series A: Biomedical Sciences and Medical Sciences 69 (8):1040-1048
Elison E, Vigsnaes LK, Krogsgaard LR, Rasmussen J, Sørensen N, McConnell B, Hennet T, Sommer MO, Bytzer P (2016) Oral supplementation of healthy adults with 2'-O-fucosyllactose and lacto-N-neotetraose is well tolerated and shifts the intestinal microbiota. British Journal of Nutrition 116 (8):1356-1368
Espín JC, Larrosa M, García-Conesa MT, Tomás-Barberán F (2013) Biological significance of urolithins, the gut microbial ellagic Acid-derived metabolites: the evidence so far. Evid Based Complement Alternat Med 2013:270418-270418. doi:10.1155/2013/270418
Fang EF, Hou Y, Palikaras K, Adriaanse BA, Kerr JS, Yang B, Lautrup S, Hasan-Olive MM, Caponio D, Dan X (2019) Mitophagy inhibits amyloid-β and tau pathology and reverses cognitive deficits in models of Alzheimer's disease. Nature neuroscience 22 (3):401
Ferri E, Marzetti E, Calvani R, Picca A, Cesari M, Arosio B (2020) Role of Age-Related Mitochondrial Dysfunction in Sarcopenia. Int J Mol Sci 21 (15):5236. doi:10.3390/ijms21155236
García-Mantrana I, Calatayud M, Romo-Vaquero M, Espín JC, Selma MV, Collado MC (2019) Urolithin metabotypes can determine the modulation of gut microbiota in healthy individuals by tracking walnuts consumption over three days. Nutrients 11 (10):2483
Garcia-Villalba R, Vissenaekens H, Pitart J, Romo-Vaquero M, Espín JC, Grootaert C, Selma MV, Raes K, Smagghe G, Possemiers S (2017) Gastrointestinal simulation model TWIN-SHIME shows differences between human urolithin-metabotypes in gut microbiota composition, pomegranate polyphenol metabolism, and transport along the intestinal tract. Journal of agricultural and food chemistry 65 (27):5480-5493
González-Sarrías A, García-Villalba R, Romo-Vaquero M, Alasalvar C, Orem A, Zafrilla P, Tomás-Barberán FA, Selma MV, Espín JC (2017) Clustering according to urolithin metabotype explains the interindividual variability in the improvement of cardiovascular risk biomarkers in overweight-obese individuals consuming pomegranate: A randomized clinical trial. Molecular nutrition & food research 61 (5):1600830
Haas RH (2019) Mitochondrial Dysfunction in Aging and Diseases of Aging. Biology 8 (2). doi:https://doi.org/10.3390/biology8020048
Li Z, Henning SM, Lee R-P, Lu Q-Y, Summanen PH, Thames G, Corbett K, Downes J, Tseng C-H, Finegold SM (2015) Pomegranate extract induces ellagitannin metabolite formation and changes stool microbiota in healthy volunteers. Food & function 6 (8):2487-2495
Nuñez-Sánchez MA, García-Villalba R, Monedero-Saiz T, García-Talavera NV, Gómez-Sánchez MB, Sanchez-Alvarez C, García-Albert AM, Rodriguez-Gil FJ, Ruiz-Marín M, Pastor-Quirante FA (2014) Targeted metabolic profiling of pomegranate polyphenols and urolithins in plasma, urine and colon tissues from colorectal cancer patients. Molecular nutrition & food research 58 (6):1199-1211
Romo-Vaquero M, García-Villalba R, González-Sarrías A, Beltrán D, Tomás-Barberán FA, Espín JC, Selma MV (2015) Interindividual variability in the human metabolism of ellagic acid: Contribution of Gordonibacter to urolithin production. Journal of Functional Foods 17:785-791
Romo-Vaquero M, Cortés-Martín A, Loria-Kohen V, Ramírez-de-Molina A, García-Mantrana I, Collado MC, Espín JC, Selma MV (2019) Deciphering the human gut microbiome of urolithin metabotypes: association with enterotypes and potential cardiometabolic health implications. Molecular nutrition & food research 63 (4):1800958
Ryu D, Mouchiroud L, Andreux PA, Katsyuba E, Moullan N, Nicolet-dit-Felix AA, Williams EG, Jha P, Sasso GL, Huzard D (2016) Urolithin A induces mitophagy and prolongs lifespan in C. elegans and increases muscle function in rodents. Nature medicine 22 (8):879
Selma MV, González-Sarrías A, Salas-Salvado J, Andrés-Lacueva C, Alasalvar C, Orem A, Tomás-Barberán FA, Espín JC (2018) The gut microbiota metabolism of pomegranate or walnut ellagitannins yields two urolithin-metabotypes that correlate with cardiometabolic risk biomarkers: Comparison between normoweight, overweight-obesity and metabolic syndrome. Clinical Nutrition 37 (3):897-905
Selma MV, Romo-Vaquero M, Garcia-Villalba R, González-Sarrías A, Tomás-Barberán FA, Espín JC (2016) The human gut microbial ecology associated with overweight and obesity determines ellagic acid metabolism. Food & function 7 (4):1769-1774
Srivastava S (2017) The mitochondrial basis of aging and age-related disorders. Genes 8 (12):398
Tomas-Barberan FA, García-Villalba Ro, Gonzalez-Sarrias A, Selma MV, Espin JC (2014) Ellagic acid metabolism by human gut microbiota: consistent observation of three urolithin phenotypes in intervention trials, independent of food source, age, and health status. Journal of agricultural and food chemistry 62 (28):6535-6538
Tomás-Barberán FA, González-Sarrías A, Garcia-Villalba R, Nuñez-Sánchez MA, Selma MV, Garcia-Conesa MT, Espín JC (2017) Urolithins, the rescue of "old" metabolites to understand a "new" concept: Metabotypes as a nexus among phenolic metabolism, microbiota dysbiosis, and host health status. Molecular nutrition & food research 61 (1):1500901
Truchado P, Larrosa M, Garcia-Conesa MT, Cerdá Ba, Vidal-Guevara ML, Tomás-Barberán FA, Espín JC (2012) Strawberry processing does not affect the production and urinary excretion of urolithins, ellagic acid metabolites, in humans. Journal of Agricultural and Food Chemistry 60 (23):5749-5754

## Claims

1. A composition comprising:
a. a source of ellagitannins, and
b. one or more human milk oligosaccharides (HMOs).

2. A composition according to claim 1 wherein the source of ellagitannins is an extract of the husk of *Punica granatum.*

3. A composition according to claim 1 wherein the source of ellagitannins is a preparation of the fruit of *Muscodinia rotundifolia* or a preparation of the fruit of *Juglans regia.*

4. A composition according to any one of claim 1-3 wherein the HMO is selected from neutral HMOs or acidic HMOs.

5. A composition according to any one of claim 4 wherein the HMO is one or more fucosylated HMOs or one or more non-fucosylated HMOs, or a mixture of fucosylated and non-fucosylated HMOs.

6. A composition according to any one of claim 1-5 wherein the one or more HMOs is selected from 2'-FL, 3-FL, DFL, LNT, LNnT, 3'-SL, 6'-SL, LNFP-1 or a mixture thereof.

7. A composition according to claim 6 wherein the one or more HMOs comprises, consists of or essentially consists of:
a. 2'-FL and at least one of LNnT and LNT;
b. at least one of 2'-FL and DFL and at least one of LNnT and LNT;
c. 2'FL and 6'-SL;
d. DFL and 6'-SL;
e. 2'-FL, DFL and 6'-SL;
f. 2'-FL, 6'-SL and at least one of LNnT and LNT; or
g. 2'-FL, DFL, 6'-SL and at least one of LNnT and LNT.

8. A composition according to any one of claim 1-7 which contains an extract of the husk of *Punica granatum* and one or more human milk oligosaccharides (HMOs) in a ratio (w/w) of from 5:1 to 1:20.

9. A composition according to any one of claim 1-8 formulated as a single preparation.

10. A composition according to any one of claim 1-8 formulated as a kit comprising
a. a source of ellagitannins, such as an extract of the husk of *Punica granatum,* and
b. one or more human milk oligosaccharides (HMOs),
as separate components, together with instructions for use.

11. A composition according to any one of claim 1-10 for use in the treatment of a human subject identified as being a mixed urolithin producer, wherein said use comprises an oral administration of said composition in a daily dose containing between 500 - 10000 mg HMOs/day and 500 - 5000 mg of an extract of the husk of *Punica granatum*/*day.*

12. A composition according to claim 11 for use in the treatment of a human subject identified as being a mixed urolithin producer, wherein said use comprises an oral administration of said composition, containing a dosage of at least 75 mg punicalagins/day, such as between 75 mg/day and 1500 mg/day, such as between 100 mg/day and 1000 mg/day, preferably at least 300 mg/day.

13. A composition according to any one of claim 11 or 12 for use in the treatment of a human subject identified as being a mixed urolithin producer, wherein said use comprises an oral administration of said composition, containing between 500 mg and 10000 mg HMOs/day, such as at least 500 mg HMOs/day, such as at least 750 mg HMOs/day, such as at least 1000 mg HMOs/day, such as at least 2000 mg/day such as at least 5000 mg HMOs/day.

14. A method for altering the metabotype in a human subject from a mixed producer to a UA-producer, wherein a mixed producer is identified by
a. Orally administering to the human subject a standardized dose of ellagitannins for seven consecutive days,
b. On the eighth day after the human subject has ingested the first standardized dose of ellagitannins, obtaining a urine sample from the human subject,
c. Analyzing the urine sample for the presence and levels of urolithin A, urolithin B and isourolithin A,
d. Establishing the levels of urolithin A, urolithin B and isourolithin A,
e. Determining whether the subject is a UA-producer, mixed producer or non-producer, based on the comparison of the subject levels with relevant reference levels,
wherein the mixed producer metabotype is defined by having a total amount of either urolithin B or isourolithin A which is at least 50% of the total amount of urolithin A in said urine sample,
and wherein said method comprises administering a composition according to any one of claims 1-13 to said human subject for a period of at least 2 weeks.

15. A method for increasing the production of urolithins, in particular urolithin A metabolites, in the human gut, as measured in plasma and/or urine, said method comprising administering a composition according to any one of claims 1-13 to a human subject, in particular a middle-aged or elderly (40+) human subject and in particular a human subject of the mixed producer metabotype according to claim 14.
